# FASCICULE DE BREVET EUROPEEN

(11) **EP 1 090 017 B1**
(45) Date de publication et mention de la délivrance du brevet: **30.10.2002**
(21) Numéro de dépôt: 99923721.7
(22) Date de dépôt: 11.06.1999
(51) Int. Cl.: C07H 15/203, A61K 31/70

(54) **NOUVEAUX COMPOSES DERIVES DE ALPHA-D-XYLOSE, PREPARATION ET UTILISATION EN THERAPEUTIQUE**
ALPHA-D-XYLOSE VERBINDUNGEN, HERSTELLUNG UND VERWENDUNG ALS ARZNEIMITTELN
NOVEL COMPOUNDS DERIVED FROM ALPHA-D-XYLOSE, PREPARATION METHOD AND THERAPEUTIC USE

(30) Priorité: 24.06.1998 US 90566 P
(43) Date de publication de la demande: 11.04.2001
(73) Titulaire: FOURNIER INDUSTRIE ET SANTE, 21300 Chenôve (FR)
(72) Inventeur: BARBEROUSSE, Véronique, F-21121 Hauteville-Les-Dijon (FR); LEGENDRE, Christiane, F-21370 Velars-Sur-Ouche (FR); SAMRETH, Soth, F-21121 Daix (FR); EDGAR, Alan, Dunlap, F-21490 Saint-Julien (FR)
(74) Mandataire: Hubert, Philippe
(86) Numéro de dépôt international: FR9901387
(87) Numéro de publication internationale: WO99067261

(56) Documents cités:
- EP-A- 0 051 023
- EP-A- 0 290 321
- F.BELLAMY ET AL.: "Glycosylated Derivatives of Benzophenone, Benzhydrol, and Benzhydril as Potential Venous Antithrombotic Agents." JOURNAL OF MEDICINAL CHEMISTRY., vol. 36, no. 7, 2 avril 1993 (1993-04-02), pages 898-903, XP002111914 AMERICAN CHEMICAL SOCIETY. WASHINGTON., US ISSN: 0022-2623

## Description

### Domaine de l'invention

La présente invention concerne, en tant que produits industriels nouveaux des dérivés de α-D-xylose définis par la formule I ci-après. Elle concerne également le procédé de préparation de ces composés, ainsi que les compositions thérapeutiques les contenant au titre d'ingrédients actifs.

### Art antérieur

On connaît déjà, par exemple selon EP-A-0051023, des dérivés du β-D-xylose, notamment des dérivés de benzoyl- ou de α-hydroxy-benzyl-phényl β-D-xylosides, préconisés en thérapeutique pour traiter les thromboses veineuses.

On connaît également, selon EP-A-0133103, des dérivés de benzyl-phényl β-D-xylosides, présentant une activité hypocholestérolémiante et/ou hypolipidémiante.

On connaît aussi, selon EP-A-0365397, EP-A-0290321, EP-A-0133 103 et EP-A-0051023 des dérivés de type β-D-phénylthioxylosides, utiles pour leur activité antithrombotique.

L'activité antithrombotique d'un certain nombre de dérivés du β-D-xylose a également été rapportée et étudiée dans l'article de J. Med. Chem., 1993, 36 (No. 7) pages 898-903. Les recherches entreprises au laboratoire ont démontré que ces composés dérivés du β-D-xylose, étaient de bons substrats de la galactosyltransférase I. De ce fait, ces composés, actifs par voie orale, initient la synthèse des glycosaminoglycanes (GAGs). Après administration par voie orale des composés, les taux circulants de GAGs sont sensiblement augmentés et environ 20 % de ceux-ci présentent une activité de type dermatan-sulfate capable d'inhiber la thrombine, via HC II (Héparine Cofacteur II), cette initiation de la biosynthèse des GAGs étant probablement responsable de l'activité antithrombotique observée expérimentalement pour les composés précédemment évoqués. En corrélation avec le potentiel de ces composés à réduire la formation des thromboses veineuses, seuls les dérivés de configuration β du D-xylose augmentent la synthèse des GAGs. Ainsi, par exemple, l'article de MASSON et al., J. Biol. Chem., 1995, 270 (N.6), pages 2662-2668, étudie en détail le mode d'action du naroparcil, i.e. [4-(4-cyanobenzyl)phényl]-1,5-dithio-β-D-xylopyranoside, en indiquant son affinité particulière pour les GAGs alors que l'isomère α-D, cité pour comparaison sans autres précisions quant à son identification et sa préparation, est inactif vis-à-vis des GAGs. Les autres dérivés de type glycopyranoside se sont montrés inactifs dans ce domaine, aussi bien d'un point de vue biologique sur la synthèse des GAGs, que d'un point de vue pharmacologique sur la réduction ou la prévention des thromboses veineuses.

L'activité de certains dérivés du β-D-xylose, notamment l'estradiol β-D-xyloside, a été étudiée dans la publication Journal of Biological Chemistry, 1991, 266 (No. 11) pages 6674-6677 et les auteurs établissent une relation entre ce composé et la biosynthèse de l'héparane sulfate, ainsi qu'un rôle d'initiateur du β-D-xyloside dans la synthèse de chondroïtine sulfate. Ces études confirment l'intérêt des dérivés du β-D-xylose pour le traitement ou la prévention des thromboses veineuses.

### But de l'invention

Selon l'invention, on se propose de fournir une nouvelle solution technique permettant d'aboutir à des produits nouveaux biologiquement intéressants vis-à-vis des plaques athéromateuses artérielles.

### Objet de l'invention

Selon la nouvelle solution technique de l'invention, on fait appel à des produits du type α-D-xylose ou α-D-thioxylose.

On a en effet trouvé, de façon surprenante, que des dérivés du D-xylose ou du 5-thio-D-xylose présentant non plus la configuration β, mais la configuration α sur le carbone anomère, possèdent une activité particulièrement intéressante pour la prévention ou la régression des plaques athéromateuses artérielles.

Les nouveaux produits selon l'invention, qui sont des composés de α-D-xylose, sont caractérisés en ce qu'ils sont choisis parmi les composés de formule générale I : 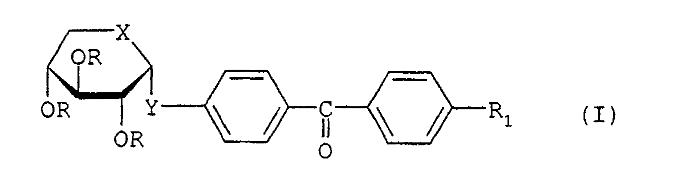 dans laquelle
X et Y représentent, indépendamment l'un de l'autre, un atome d'oxygène ou un atome de soufre,
R₁ représente un groupe CN, CF₃ ou SO₂CH₃, et
R représente un atome d'hydrogène ou un groupe acyle aliphatique comprenant 2 à 5 atomes de carbone.

Selon un autre aspect de l'invention, l'on fournit une composition pharmaceutique caractérisée en ce qu'elle renferme, en association avec un excipient physiologiquement acceptable, une quantité thérapeutiquement efficace d'au moins un composé de formule I.

L'on préconise également l'utilisation d'un composé de formule I en tant que médicament antiathéromateux.

### Description détaillée de l'invention

Comme présentés dans la formule I ci-dessus, les composés selon l'invention sont des dérivés de α-D-xylose ou de α-D-thioxylose, substitués sur le carbone anomère en position α par un groupement benzophénone substitué.

Les fonctions hydroxyle du D-xylose ou D-thioxylose peuvent être libres ou substituées par un groupement acyle comprenant 2 à 5 atomes de carbone, préférentiellement le groupe acétyle.

Par groupe acyle aliphatique comprenant 2 à 5 atomes de carbone, on entend ici un groupe acyle à chaîne linéaire, ramifiée ou cyclisée, tel que notamment CH₃CO, CH₃CH₂CO, (CH₃)₂CHCO, (CH₃)₃CCO, ou cyclopropylcarbonyle.

Les composés de formule I peuvent être préparés selon une méthode connue en soi par application de mécanismes réactionnels classiques. Selon un mode opératoire préféré on fait réagir (selon une réaction de couplage) une benzophénone de formule II : 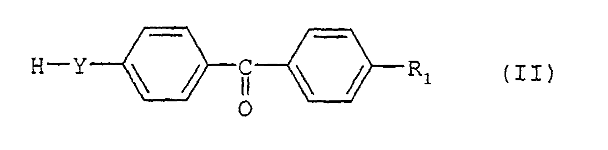 dans laquelle Y est l'atome d'oxygène ou l'atome de soufre et R₁ représente CN, CF₃ ou SO₂CH₃,
avec un composé de D-xylose (ou de 5-thio-D-xylose) de formule III : 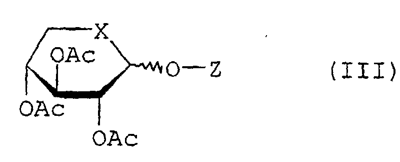 dans laquelle 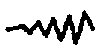 représente une liaison de configuration indéterminée (α, β ou mélange α/β), X est un atome d'oxygène ou un atome de soufre, Ac représente le groupe acétyle, Z représente un groupe acétyle ou un groupe trichlorométhylimino [-C(=NH)-CCl₃], la réaction étant conduite dans un solvant et en présence d'un acide de Lewis, pour obtenir après purification un composé de formule I : 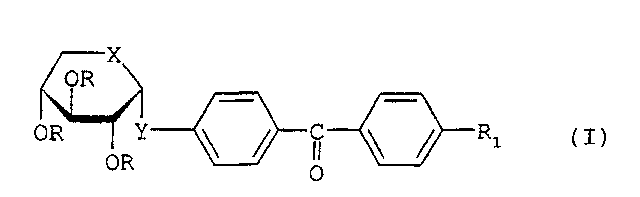 dans laquelle
X, Y, et R₁ conservent la même signification que dans les produits de départ et R représente un groupe acétyle.

Les composés de formule I dans lesquels R est un atome d'hydrogène peuvent être obtenus à partir des composés précédents, dans lesquels R est le groupe acétyle, par action d'une base telle que par exemple le méthylate de sodium ou l'ammoniac qui permettent de remplacer le groupe acétyle par un atome d'hydrogène.

Les composés de formule I dans lesquels R est un groupe acyle en C₂-C₅, tel que défini ci-dessus, peuvent être obtenus à partir des composés de formule I dans lesquels R est un atome d'hydrogène, par action du chlorure ou de l'anhydride d'acide correspondant au groupe acyle en C₂-C₅ souhaité, en présence d'une base aprotique telle que par exemple la triéthylamine ou la pyridine, et dans un solvant tel que par exemple le dichlorométhane.

En bref, le procédé de préparation selon l'invention comprend (a) la réaction de couplage II + III et la purification du composé I (R = Ac) ainsi obtenu, (b) si nécessaire l'hydrolyse (saponification) dudit composé de formule I (R = Ac) pour obtenir le composé de formule I désacétylé (R = H), et (c) si nécessaire l'estérification dudit composé de formule I (R = H) pour obtenir tout autre composé de formule I estérifié (R = acyle, notamment un groupe acyle différent de Ac).

Les composés de formule I sont utiles en thérapeutique en tant que principes actifs de médicaments destinés au traitement ou à la prévention de l'athérosclérose.

Selon l'invention, on préconise l'utilisation d'un produit choisi parmi l'ensemble constitué par les composés de formule I ci-dessus pour la préparation d'un médicament antiathéromateux destiné à une utilisation en thérapeutique vis-à-vis de l'athérosclérose.

Les produits préférés selon l'invention, eu égard à leurs propriétés bénéfiques vis-à-vis de l'athérosclérose, sont les composés de formule I où R₁ est CN, et parmi ceux-ci les produits dans lesquels X = Y = S ou X = Y = O.

Les exemples de préparation qui suivent, non limitatifs, permettent de mieux comprendre et apprécier les avantages de l'invention.

### Exemple 1

### [4-(4-cyanobenzoyl)phényl] 2,3,4-tri-O-acétyl-1,5-dithio-α-D-xylopyranoside

On prépare une suspension de 5 g (15.10⁻³ mole) de tétra-O-acétyl-5-thio-D-xylopyranose, 4,3 g (18.10⁻³ mole) de 4-(4-mercaptobenzoyl)benzonitrile et 5 g de tamis moléculaire 4 Å dans 100 ml d'acétonitrile et on ajoute, à 0°C et sous agitation, 4 ml d'éthérate du trifluorure de bore (à 48 %, d = 1,13). On laisse le mélange réactionnel revenir à température ambiante et on agite pendant une heure. Le mélange est ensuite filtré puis concentré sous pression réduite. Le résidu est repris à l'acétate d'éthyle et la solution est lavée par de la soude diluée puis par de l'acide chlorhydrique dilué et enfin par de l'eau jusqu'à neutralité. La phase organique est séchée et concentrée sous pression réduite. Le résidu est purifié par chromatographie sur gel de silice en éluant par le mélange toluène/acétate d'éthyle (6/1 ; v/v). On obtient ainsi 7 g du composé attendu en mélange avec l'isomère β (les deux isomères sont dans le rapport α/β = 60/40). Ce mélange est dissout dans 15 ml d'acétate d'éthyle et on ajoute 15 ml d'éther éthylique. Les cristaux obtenus (essentiellement l'isomère β) sont éliminés par filtration et le filtrat est concentré sous pression réduite. On obtient ainsi 5,4 g du composé attendu contenant 20 % d'isomère β (rendement = 63 %). Le composé attendu est obtenu à 98 % de pureté en isomère α après deux recristallisations dans le méthanol.
F = 144-146°C
[α]²⁴_{D} = + 328° (c = 0,42 ; CH₂Cl₂)

### Exemple 2

### [4-(4-cyanobenzoyl)phényl] 1,5-dithio-α-D-xylopyranoside

On prépare une solution de 10,27 g (20.10⁻³ mole) du composé obtenu selon l'exemple 1, dans 100 ml de méthanol et 35 ml de tétrahydrofuranne. On ajoute ensuite, à 10-15°C, 1,15 ml (4.10⁻³ mole) d'une solution de méthylate de sodium. Après 20 mn sous agitation à 10-15°C, le mélange réactionnel est percolé sur une résine IR 120 (H⁺). La solution est décolorée par du charbon actif, filtrée et concentrée sous pression réduite. Le résidu est cristallisé dans le méthanol. Après une recristallisation dans le méthanol on obtient 5 g du produit attendu sous forme de cristaux blancs (rendement = 65 %).
F = 142°C
[α]_{D}²⁰ = + 508° (c = 0,42 ; CH₃OH)

### Exemple 3

### [4-(4-cyanobenzoyl)phényl]2,3,4-tri-O-acétyl-α-D-xylopyranoside

On prépare un mélange de 9 g (28,3.10⁻³ mole) de tétra-O-acétyl-D-xylose et 8 g (35,9.10⁻³ mole) de 4-(4-hydroxybenzoyl)benzonitrile dans 90 ml de dichlorométhane. On ajoute ensuite goutte à goutte, à température ambiante, 8 ml (68,4.10⁻³ mole) de tétrachlorure d'étain, puis on porte le milieu réactionnel à 45°C, pendant 15 heures, sous agitation. Le mélange est ensuite versé sur de la glace et de l'acide chlorhydrique dilué ; on extrait à l'acétate d'éthyle en milieu acide, puis les phases organiques rassemblées sont lavées avec une solution diluée d'acide chlorhydrique, puis à l'eau, avec une solution diluée de soude, puis à l'eau. La phase organique est séchée sur sulfate de magnésium puis concentrée sous pression réduite. Le produit brut est purifié par chromatographie sur gel de silice en éluant à l'aide d'un mélange toluène/acétate d'éthyle (5/1 ; v/v). On obtient ainsi 3,3 g du produit attendu sous forme d'une poudre blanche (rendement = 24 %).
F = 75°C
[α]²⁶_{D} = + 146,3° (c = 0,36 ; CH₃OH)

### Exemple 4

### [4-(4-cyanobenzoyl)phényl] α-D-xylopyranoside

On ajoute 4,3 g (8,94. 10⁻³ mole) du composé obtenu selon l'exemple 3 à 50 ml d'une solution saturée d'ammoniac dans le méthanol, à 0°C et on maintient le mélange sous agitation pendant 3 heures à 0°C, puis 2 heures à température ambiante. Le milieu réactionnel est ensuite concentré sous pression réduite et le résidu est purifié par chromatographie sur gel de silice en éluant avec un mélange dichlorométhane/méthanol (10/1 ; v/v). La fraction pure obtenue est concentrée pour éliminer les solvants, puis le produit est mis en suspension dans de l'eau distillée ; le mélange est congelé et lyophilisé. On obtient ainsi 2,15 g du produit attendu sous forme d'une poudre blanc cassé (rendement = 67 %).
F = 186-187°C
[α]²⁶_{D} = + 155°(c = 0,40 ; CH₃OH)

### Exemple 5

### [4-(4-cyanobenzoyl)phényl] 2,3,4-tri-O-acétyl-1-thio-α-D-xylopyranoside

En opérant de façon analogue à l'exemple 3, au départ de 4-(4-mercaptobenzoyl)benzonitrile, on obtient le produit attendu sous forme d'un solide blanc (rendement = 11 %).
F = 158-159°C
[α]²⁶_{D} = + 161° (c = 0,38 ; CH₂Cl₂)

### Exemple 6

### [4-(4-cyanobenzoyl)phényl] 1-thio-α-D-xylopyranoside

En opérant de façon analogue à l'exemple 4, au départ du composé obtenu selon l'exemple 5, on obtient le produit attendu sous forme d'une poudre crème (rendement = 90 %).
F = 198°C
[α]²⁶_{D} = + 245° (c = 0,30 ; CH₃OH)

### Exemple 7

### [4-(4-cyanobenzoyl)phényl] 2,3,4-tri-O-acétyl-5-thio-α-D-xylopyranoside

On prépare une suspension de 2 g (9.10⁻³ mole) de 4-(4-hydroxybenzoyl)benzonitrile dans 40 ml de dichlorométhane, on ajoute 1 g de tamis moléculaire 4 Å et on refroidit le mélange à -30°C. On ajoute lentement, sous agitation, 1,8 ml (8.10⁻³ mole) de trifluorométhanesulfonate de triéthylsilyle, puis une solution de 3,9 g (9.10⁻³ mole) de trichloroacétimidate de 2,3,4-tri-O-acétyl-5-thio-α-D-xylopyranosyle dans 20 ml de dichlorométhane. Le mélange réactionnel est agité pendant 4 heures à -30°C puis pendant 16 heures à 0°C. Après neutralisation à l'aide d'une solution de collidine, le milieu réactionnel est filtré. Le filtrat est dilué dans 200 ml d'acétate d'éthyle et la phase organique obtenue est lavée avec une solution de soude diluée, puis à l'eau, à l'aide d'une solution d'acide chlorhydrique dilué, puis à l'eau et séchée sur sulfate de magnésium. Après concentration de la solution sous pression réduite, le résidu est purifié par chromatographie sur gel de silice en éluant avec un mélange méthylcyclohexane-acétate d'éthyle (5/2 ; v/v). On obtient ainsi 0,86 g du produit attendu sous forme d'une poudre beige (rendement = 19 %).
F = + 85°C
[α]²¹_{D} = + 365° (c = 0,3 ; CH₂Cl₂)

### Exemple 8

### [4-(4-cyanobenzoyl)phényl] 5-thio-α-D-xylopyranoside

En opérant de façon analogue à l'exemple 4, au départ du composé obtenu selon l'exemple 7, on obtient le produit attendu sous forme d'une poudre beige claire (rendement = 73 %).
F = 180°C
[α]²⁴_{D} = + 476° (c = 0,32 ; CH₃OH)

### Exemple 9

### [4-[4-(trifluorométhyl)benzoyl]phényl] 2,3,4-tri-O-acétyl-α-D-xylopyranoside

On prépare une solution de 2,5 g (9,4.10⁻³ mole) de (4-hydroxyphényl)[4-(trifluorométhyl)phényl]méthanone et 3,5 g (11.10⁻³ mole) de tetra-O-acétyl-D-xylose dans 30 ml d'acétonitrile et on ajoute 1 g de tamis moléculaire 4 Å. On refroidit le mélange à 0°C et on ajoute goutte à goutte, sous agitation 7,2 ml (58.10⁻³ mole) d'éthérate de trifluorure de bore. On maintient ensuite le milieu réactionnel sous agitation à température ambiante pendant 15 heures, puis on filtre. Le filtrat est dilué avec de l'acétate d'éthyle et la phase organique est lavée avec une solution de soude diluée, à l'eau, avec une solution d'acide chlorhydrique dilué et à nouveau à l'eau. Après séchage sur sulfate de magnésium, la phase organique est concentrée sous pression réduite et le résidu est purifié par chromatographie sur gel de silice en éluant à l'aide d'un mélange méthylcyclohexane-acétate d'éthyle (5/2 ; v/v), on obtient ainsi 0,46 g du produit attendu sous forme d'un solide beige amorphe (rendement = 10 %).
F= 65°C
[α]²⁶_{D} = + 141° (c = 0,32 ; CH₂Cl₂)

### Exemple 10

### [4-[4-(trifluorométhyl)benzoyl]phényl] α-D-xylopyranoside

On dissout 0,370 g (0,7.10⁻³ mole) du composé obtenu selon l'exemple 9 dans 20 ml de méthanol et on ajoute 40 µl d'une solution de méthylate de sodium à 25 % dans le méthanol. La solution est maintenue pendant 2 heures sous agitation, puis on ajoute environ 0,5 g de résine IR 120 sous forme acide. Après filtration, le filtrat est concentré sous pression réduite. On obtient ainsi 240 mg du produit attendu sous forme d'un solide beige (rendement = 85 %).
F = 188°C
[α]²¹_{D} = + 165° (c = 0,27 ; CH₃OH)

### Exemple 11

### [4-[4-(trifluorométhyl)benzoyl]phényl] 2,3,4-tri-O-acétyl-5-thio-α-D-xylopyranoside

En opérant de façon analogue à l'exemple 7, au départ de (4-hydroxyphényl)[4-(trifluorométhyl)phényl]méthanone, on obtient le produit attendu sous forme d'un solide amorphe beige (rendement = 19,5 %).
F = 65°C
[α]_{D} = + 320° (c = 0,34 ; CH₂Cl₂)

### Exemple 12

### [4-[4-(trifluorométhyl)benzoyl]phényl] 5-thio-α-D-xylopyranoside

En opérant de façon analogue à l'exemple 10, au départ du composé obtenu selon l'exemple 11, on obtient le produit attendu sous forme d'une poudre blanche amorphe (rendement = 80 %).
F = 82°C
[α]²²_{D} = + 378° (c = 0,25; CH₃OH)

### PREPARATION I

### Acide diméthylcarbamothioïque, O-[4-[4-(trifluorométhyl)benzoyl]phényl] ester

On prépare une solution de 10 g (37,6.10⁻³ mole) de (4-hydroxyphényl)[4-(trifluorométhyl)phényl]méthanone dans 100 ml d'acétone et on ajoute lentement sous agitation et à température ambiante, une solution de 2,94 g (45.10⁻³ mole) d'hydroxyde de potassium (à 80 %) dans 80 ml d'eau. On ajoute ensuite une solution de 5,11 g (41,3.10⁻³ mole) de chlorure de diméthylthiocarbamoyle dans 70 ml d'acétone. Le mélange réactionnel est maintenu sous agitation pendant 8 heures à température ambiante, puis concentré sous pression réduite. Le produit brut est mis en suspension dans 60 ml d'une solution d'hydroxyde de potassium à 1 mole/l et placé sous agitation pendant 20mm à 10-15°C. Le solide est filtré, rincé en suspension avec de l'eau jusqu'à pH neutre puis séché au dessiccateur. On obtient ainsi 10,3 g du produit attendu sous forme d'une poudre beige (rendement = 78 %).
F = 174°C

### PREPARATION II

### Acide diméthylcarbamothioïque, S-[4-[4-(trifluorométhyl)benzoyl]phényl] ester

On place 10,3 g (29.10⁻³ mole) du composé obtenu selon la préparation I dans un ballon, sous atmosphère d'azote et on maintient le produit à 250-260°C pendant 1 heure. Après refroidissement, on ajoute environ 15 ml d'acétate d'éthyle, on porte le mélange à léger reflux puis on laisse refroidir jusqu'à 0°C. Après environ 10 heures à cette température, on filtre le solide cristallisé et on rince le solide avec environ 10 ml de cyclohexane. Après séchage au dessiccateur, on obtient 6,2 g du produit attendu sous forme de cristaux beiges (rendement = 60 %).
F = 140°C

### PREPARATION III

### (4-mercaptophényl)-[4-(trifluorométhyl)phényl]méthanone

On prépare une suspension de 6,1 g (17,3.10⁻³ mole) du composé obtenu selon la préparation II dans 65 ml de méthanol. Après avoir désoxygéné le milieu par barbotage d'azote, on ajoute 10 ml (soit 34.10⁻³ mole) d'une solution de méthylate de sodium dans le méthanol, puis on porte le mélange réactionnel à 40°C sous agitation pendant 3 heures. On concentre ensuite partiellement (on élimine environ 25 ml de méthanol) sous pression réduite et on verse la solution dans un mélange de glace et d'acide chlorhydrique dilué. Le produit attendu précipite. On sépare le produit solide par filtration et on le lave avec de l'eau jusqu'à pH neutre. Après séchage au dessiccateur, on obtient 4,8 g du produit attendu sous forme d'un solide vert clair.
F = 150°C

### Exemple 13

### [4-[4-(trifluorométhyl)benzoyl]phényl] 1-thio-2,3,4-tri-O-acétyl-α-D-xylopyranoside

En opérant de façon analogue à l'exemple 9, au départ du composé obtenu selon la préparation III, on obtient le produit attendu sous forme d'un solide beige (rendement = 27 %).
F = 60°C
[α]²²_{D} = + 145° (c = 0,25 ; CH₂Cl₂)

### Exemple 14

### [4-[4-(trifluorométhyl)benzoyl]phényl] 1-thio-α-D-xylopyranoside

En opérant de façon analogue à l'exemple 10, au départ du composé obtenu selon l'exemple 13, on obtient le produit attendu sous forme d'un solide amorphe crème (rendement = 95 %).
F = 172°C
[α]²⁴_{D} = + 211° (c = 0,35 ; CH₃OH)

### Exemple 15

### [4-[4-(trifluorométhyl)benzoyl]phényl] 2,3,4-tri-O-acétyl-1,5-dithio-α-D-xylopyranoside

En opérant de façon analogue à l'exemple 9, au départ de 1,2,3,4-tétra-O-acétyl-5-thio-D-xylose et du composé obtenu selon la préparation III, on obtient le produit attendu sous forme d'une poudre blanche (rendement = 18 %).
F = 70°C
[α]²⁷_{D} = + 215° (c = 0,45 ; CH₂Cl₂)

### Exemple 16

### [4-[4-(trifluorométhyl)benzoyl]phényl] 1,5-dithio-α-D-xylopyranoside

En opérant de façon analogue à l'exemple 10, au départ du composé obtenu selon l'exemple 15, on obtient le produit attendu sous forme d'un solide poudreux blanc (rendement = 84 %).
F = 104°C
[α]²⁵_{D} = + 399° (c = 0,50 ; DMSO)

### PREPARATION IV

### (4-hydroxyphényl)[4-(méthylsulfinyl)phényl]méthanone

On prépare une solution de 15 g (61,4.10⁻³ mole) de (4-hydroxyphényl)[4-(méthylthio)phényl]méthanone dans 200 ml de méthanol. On refroidit jusqu'à 5°C environ à l'aide d'un bain de glace et on ajoute par fractions 14,12 g (61,4.10⁻³ mole) d'acide 3-chloro-benzènecarboperoxoïque (mCPBA) titrant 75 %. On maintient le milieu réactionnel sous agitation pendant 15 mn après la fin de l'addition et on hydrolyse sur une solution diluée de bicarbonate de sodium. On extrait à l'aide d'acétate d'éthyle et la phase obtenue est lavée à l'eau jusqu'à neutralité, séchée et concentrée sous pression réduite. On obtient ainsi 13,2 g d'un solide blanc composé du produit attendu en mélange avec de la (4-hydroxyphényl)[4-(méthylsulfonyl)phényl]méthanone.

### PREPARATION V

### (4-hydroxyphényl)[4-(méthylsulfonyl)phényl]méthanone

On prépare une suspension de 13,2 g du composé obtenu selon la préparation IV dans 150 ml de méthanol et on ajoute sous agitation, par portions, 11,7 g (50,8.10⁻³ mole) de mCPBA (titrant 75 %). On maintient le mélange réactionnel sous agitation pendant 30 minutes puis on hydrolyse sur une solution froide de bicarbonate de sodium. On extrait à l'aide d'acétate d'éthyle et la phase organique est lavée à l'eau jusqu'à neutralité, séchée puis concentrée sous pression réduite. On obtient ainsi 13,6 g du produit attendu sous forme d'un solide blanc (rendement = 97 %).
F = 144°C

### PREPARATION VI

### Acide diméthylcarbamothioïque, O-[4-[4-(méthylsulfonyl)benzoyl]phényl] ester

En opérant de façon analogue à la préparation I, au départ du composé obtenu selon la préparation V, on obtient le produit attendu sous forme d'un solide beige (rendement = 69 %).
F = 150°C

### PREPARATION VII

### Acide diméthylcarbamothioïque, S-[4-[4-(méthylsulfonyl)benzoyl]phényl] ester

En opérant de façon analogue à la préparation II, au départ du composé obtenu selon la préparation VI, on obtient le produit attendu sous forme de cristaux beiges (rendement = 91 %).
F = 172°C

### PREPARATION VIII

### (4-mercaptophényl)[4-(méthylsulfonyl)phényl]méthanone

En opérant de façon analogue à la préparation III, au départ du composé obtenu selon la préparation VII (en ajoutant de la diméthylformamide pour solubiliser le produit), on obtient le produit attendu sous forme d'un solide crème (rendement = 95 %).
F = 176°C

### Exemple 17

### [4-[4-(méthylsulfonyl)benzoyl]phényl] 2,3,4-tri-O-acétyl-1,5-dithio-α-D-xylopyranoside

En opérant de façon analogue à l'exemple 15, au départ du composé obtenu selon la préparation VIII, on obtient le produit attendu sous forme d'un solide beige (rendement = 31 %).
F = 86°C
[α]²¹_{D} = + 293° (c = 0,44 ; DMSO)

### Exemple 18

### [4-[4-(méthylsulfonyl)benzoyl]phényl] 1,5-dithio-α-D-xylopyranoside

En opérant de façon analogue à l'exemple 10, au départ du composé obtenu selon l'exemple 17, on obtient le produit attendu sous forme d'un solide jaune pâle (rendement = 90,5 %).
F = 100°C
[α]²⁶_{D} = + 381° (c = 0,58 ; DMSO)

L'activité antiathéromateuse des composés selon l'invention a été mise en évidence sur des souris femelles, déficientes en apolipoprotéine E (homozygotes). Selon le protocole de ce test, le produit à évaluer est administré dans la nourriture (régime alimentaire standard) pendant 14 semaines. Après ce laps de temps, les souris sont euthanasiées et le coeur et la crosse aortique sont prélevés. Les surfaces lésées sont évaluées selon la méthode décrite dans Arteriosclerosis, 1990, 10, p. 316-323.

Le tableau I présente les résultats obtenus selon ce test pratiqué avec le composé de l'exemple 2 à différentes doses. Les résultats sont exprimés en pourcentage de réduction de la surface des plaques d'athérome, par rapport à un groupe de souris témoin non traitées.

**Tableau I**

| | | | |
|---|---|---|---|
| Dose administrée (mg/kg) | 10 | 100 | 300 |
| Réduction (%) | - 32 | - 51 | - 71 |

A titre de comparaison, on a testé également le composé [4-(4-cyanobenzoyl)phényl] 1,5-dithio- β-D-xylopyranoside, décrit précédemment dans EP-A-0290321. A la dose de 300 mg/kg, ce composé réduit de 30 % la surface lésée. Comme présenté sur le tableau I, le même résultat biologique est obtenu avec seulement 10 mg/kg du composé selon l'exemple 2. Ceci démontre la supériorité du composé de l'invention dans cette activité lorsque le carbone anomère du xylose est en configuration α.

Durant les 14 semaines de l'essai décrit ci-dessus dans le but d'évaluer les propriétés antiathéromateuses des composés, on a mesuré périodiquement le taux de cholestérol sérique des souris traitées et des souris témoins, et le résultat est exprimé sous la forme aire sous courbe (AUC) du cholestérol pendant toute la durée de l'essai. Les résultats obtenus avec le composé de l'exemple 2 sont reportés dans le tableau II où AUC représente l'aire sous courbe (exprimée en g.jour.1⁻¹) et la variation (en pourcentage) est appréciée par rapport au groupe témoin.

**Tableau II**

| | | | | |
|---|---|---|---|---|
| dose(mg/kg) (Ex 2) | 0 (témoin) | 10 | 100 | 300 |
| AUC | 583 ± 23 | 585 ± 15 | 507 ± 16 | 505 ± 20 |
| variation (%) | - | 0 | - 13 | - 13 |

L'analyse des valeurs observées montre un effet hypocholestérolémiant qui n'apparaît sur la période de 14 semaine qu'à la dose de 100 mg/kg alors que l'effet antiathéromateux est significatif dès la dose de 10 mg/kg.

Toutefois, en raison de la probabilité d'une corrélation entre l'activité antiathéromateuse observée et une aptitude du composé à abaisser le taux de cholestérol sérique, et afin d'obtenir un résultat de screening plus rapide, les composés selon l'invention ont été évalués en fonction de leur potentiel à diminuer la cholestérolémie de souris soumises à un régime riche en graisses. Le test est réalisé par administration à des souris femelles de souche C57BL/6J. Le protocole est le suivant : le premier jour (J0), les souris sont mises à jeun de 9 à 17 heures, un prélèvement sanguin étant effectué à 14 heures. A 17 heures, une quantité déterminée de nourriture (régime gras comprenant 1,25 % de cholestérol et 0,5 % d'acide cholique) est distribuée. Le second jour (J1), à 9 heures, les restes de nourriture sont pesés et les souris mises à jeun de 9 à 14 heures. A 14 heures, un prélèvement sanguin est effectué. Pour les groupes de souris traitées, le composé est administré par tubage, en suspension dans une solution d'eau gommeuse, à 3 %, le second jour (J1) à 9 heures. Les groupes témoins reçoivent seulement de l'eau gommeuse.

Les composés ont été testés à la dose de 100 mg/kg. Le cholestérol total sérique est dosé et les résultats sont exprimés en pourcentage d'inhibition de l'augmentation de la cholestérolémie par rapport au groupe témoin. Les résultats obtenus sont reportés dans la colonne "Activité" du tableau III. Par ailleurs, on peut noter que l'analyse du contenu en cholestérol des différentes classes de lipoprotéines sériques montre un effet favorable du produit sur le rapport HDL-cholestérol/cholestérol total.

Les produits de formule I selon l'invention peuvent être administrés, de préférence par voie orale, sous forme de comprimés ou de gélules, renfermant chacun 20 à 500 mg d'un composé de formule I en tant que principe actif, en association avec des excipients. La posologie sera d'environ 1 à 4 prises par jour. Ces produits seront prescrits pour la prévention ou le traitement du risque athéromateux.

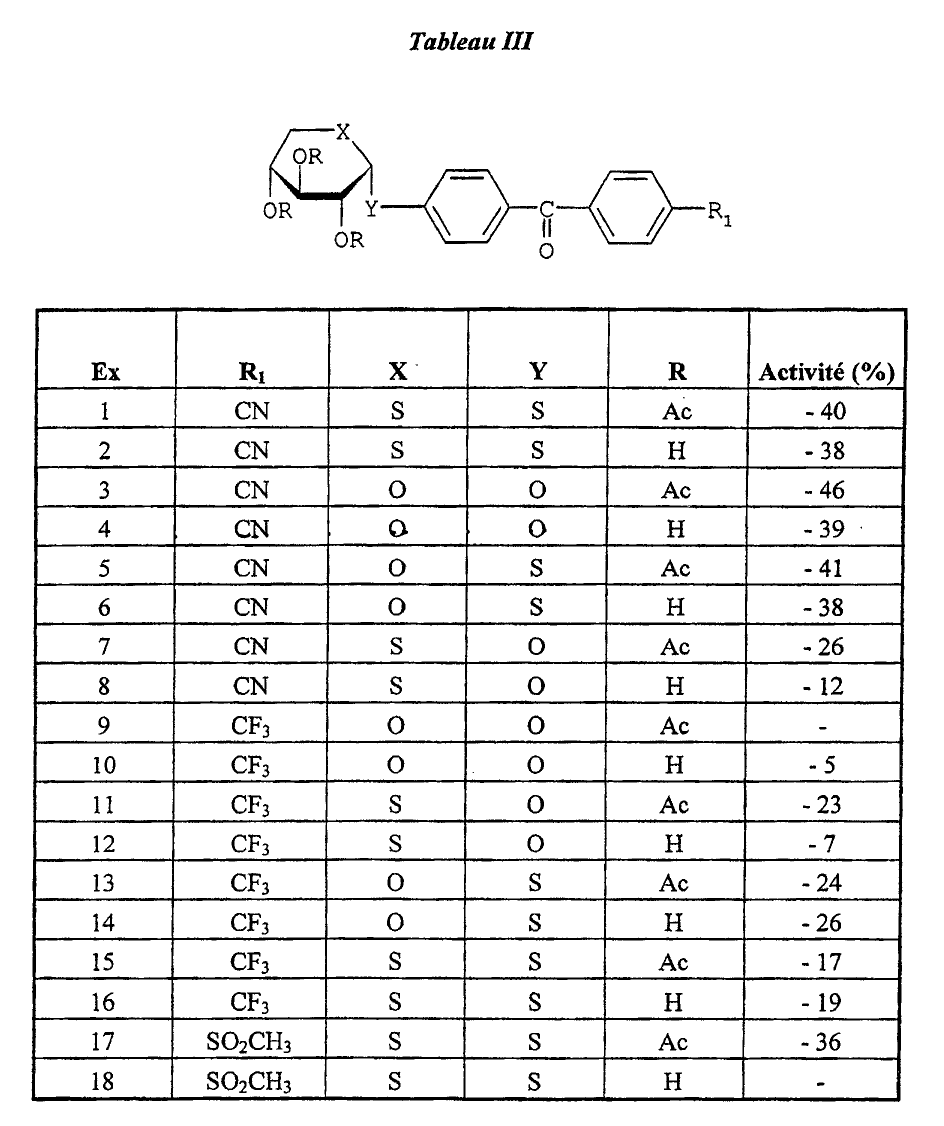

## Revendications

1. Composé de α-D-xylose **caractérisé en ce qu'**il est choisi parmi l'ensemble constitué par les composés de formule I : 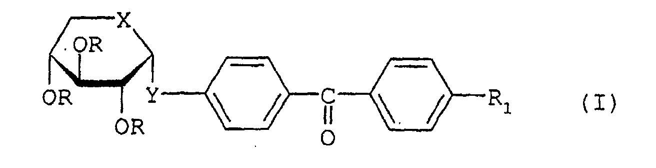 dans laquelle
X et Y représentent, indépendamment l'un de l'autre, un atome d'oxygène ou un atome de soufre,
R₁ représente un groupe CN, CF₃ ou SO₂CH₃, et
R représente un atome d'hydrogène ou un groupe acyle aliphatique comprenant 2 à 5 atomes de carbone.

2. Composé de α-D-xylose de formule I selon la revendication 1, dans lequel R₁ est CN.

3. Composé de α-D-xylose de formule I selon la revendication 1, dans lequel X = Y = S et R₁ = CN.

4. Composé de α-D-xylose de formule I selon la revendication 1, dans lequel X = Y = O et R₁ = CN.

5. Procédé de préparation d'un composé de formule I selon la revendication 1, **caractérisé en ce que**
(a) on fait réagir un composé benzophénone de formule II : 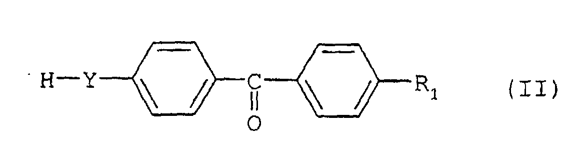 dans laquelle Y est l'atome d'oxygène ou l'atome de soufre et R₁ représente CN, CF₃ ou SO₂CH₃,
avec un composé D-xylose de formule III : 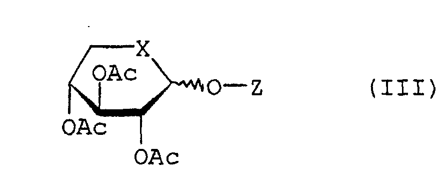 dans laquelle
X est un atome d'oxygène ou un atome de soufre,
Ac représente le groupe acétyle et Z représente un groupe acétyle ou un groupe trichlorométhylimino [-C(=NH)-CCl₃],
la réaction étant conduite dans un solvant et en présence d'un acide de Lewis, pour obtenir après purification un composé de formule I : 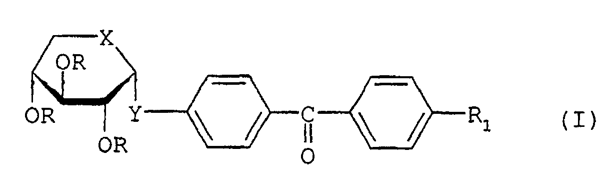 dans laquelle
X, Y, et R₁ conservent la même signification que dans les produits de départ et R représente un groupe acétyle,
(b) si nécessaire, on fait réagir le composé de formule I, ainsi obtenu et dans lequel R est le groupe acétyle, avec une base pour remplacer le groupe acétyle par un atome d'hydrogène et obtenir le composé de formule I dans lequel R est un atome d'hydrogène, et
(c) si nécessaire, on fait réagir le composé de formule I, ainsi obtenu et dans lequel R est un atome d'hydrogène, avec un chlorure d'acide en C₂-C₅ ou un anhydride d'un acide aliphatique en C₂-C₅, en présence d'une base aprotique et dans un solvant, pour obtenir le composé de formule I dans lequel R est un groupe acyle.

6. Composition pharmaceutique **caractérisée en ce qu'**elle renferme, en association avec un excipient physiologiquement acceptable, une quantité thérapeutiquement efficace d'au moins un composé de formule I selon la revendication 1.

7. Utilisation d'un produit choisi parmi l'ensemble des composés de formule I selon la revendication 1, pour la préparation d'un médicament antiathéromateux destiné à une utilisation en thérapeutique vis-à-vis de l'athérosclérose.

## Patentansprüche

1. α-D-Xyloloseverbindung, **dadurch gekennzeichnet, dass** sie aus der Gruppe ausgewählt ist, die aus den Verbindungen der Formel I besteht: 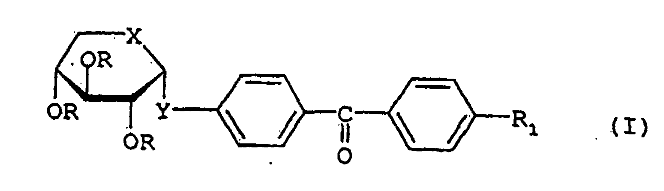 in der X und Y unabhängig voneinander ein Sauerstoffatom oder ein Schwefelatom darstellen,
R₁ eine CN, CF₃ oder SO₂CH₃ Gruppe darstellt, und
R ein Wasserstoffatom oder eine aliphatische Acylgruppe, die 2 bis 5 Kohlenstoffatome umfasst, darstellt..

2. α-D-Xyloloseverbindung der Formel I gemäß Anspruch 1, wobei R₁ CN ist.

3. α-D-Xyloloseverbindung der Formel I gemäß Anspruch 1, wobei X = Y = S und R₁ = CN ist.

4. α-D-Xyloloseverbindung der Formel I gemäß Anspruch 1, wobei X = Y = O und R₁ = CN ist.

5. Verfahren zur Herstellung einer Verbindung der Formel I gemäß Anspruch 1, **dadurch gekennzeichnet, dass**
(a) man eine Benzophenonverbindung der Formel II: 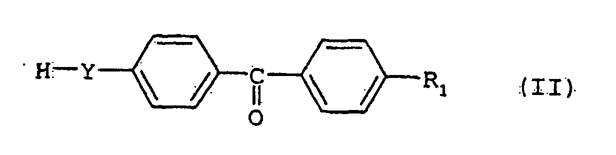 in der Y ein Sauerstoffatom oder ein Schwefelatom ist und R₁ CN, CF₃ oder SO₂CH₃ darstellt,
mit einer D-Xyloloseverbindung der Formel III umsetzt: 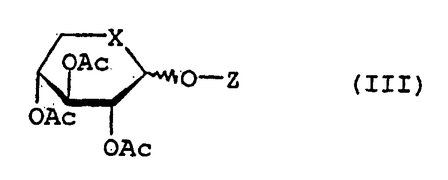 in der X ein Sauerstoffatom oder ein Schwefelatom ist,
Ac eine Acetylgruppe darstellt und Z eine Acetylgruppe oder eine Trichlormethyliminogruppe [-C(=NH)-CCl₃] darstellt,
wobei die Umsetzung in einem Lösungsmittel und in Gegenwart einer Lewis-Säure durchgeführt wird, um nach der Aufreinigung eine Verbindung der Formel I zu erhalten: 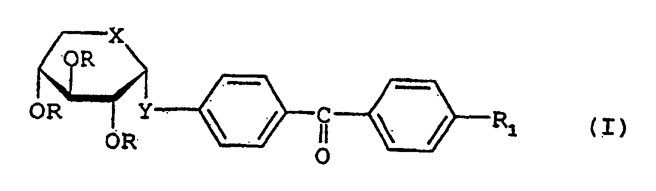 in der X, Y und R₁ die gleiche Bedeutung behalten, wie in den Ausgangsprodukten und R eine Acetylgruppe darstellt,
(b) falls nötig, lässt man die Verbindung der Formel I, die so erhalten wurde, und in der R die Acetylgruppe ist, mit einer Base reagieren, um die Acetylgruppe durch ein Wasserstoffatom zu ersetzen und die Verbindung der Formel I zu erhalten, in der R ein Wasserstoffatom ist, und
(c) falls nötig, lässt man die Verbindung der Formel I, die so erhalten wurde und in der R ein Wasserstoffatom ist, mit einer C₂-C₅-Chlorsäure oder einem Anhydrid einer aliphatischen C₂-C₅-Säure in Gegenwart einer aprotischen Base und in einem Lösungsmittel reagieren, um die Verbindung der Formel I zu erhalten, in der R eine Acylgruppe ist.

6. Pharmazeutische Zusammensetzung, **dadurch gekennzeichnet, dass** sie in Verbindung mit einem physiologisch akzeptablen Träger eine therapeutisch wirksame Menge mindestens einer Verbindung der Formel I gemäß Anspruch 1 einschließt.

7. Verwendung eines Produktes, das aus der Gruppe der Verbindungen nach Formel I gemäß Anspruch 1 ausgewählt ist, zur Herstellung eines Medikamentes gegen Atheroma, das auf eine therapeutische Verwendung bei der Arteriosklerose ausgerichtet ist.

## Claims

1. An α-D-xylose compound **characterised in that** it is selected from the group consisting of the compounds of the formula I. 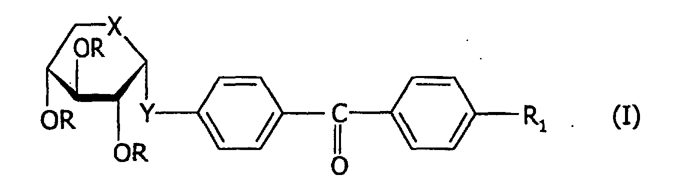 wherein
X and Y represent, independently of one another, an oxygen atom or a sulphur atom,
R₁ represents a CN, CF₃ or SO₂CH₃ group, and,
R represents a hydrogen atom or an aliphatic acyl group containing 2 to 5 carbon atoms.

2. An α-D-xylose compound of the formula I according to claim 1, in which R₁ is CN.

3. An α-D-xylose compound of the formula I according to claim 1, in which X=Y=S and R₁ is CN.

4. An α-D-xylose compound of the formula I according to claim 1, in which X = Y = O and R₁ is CN.

5. A method for preparing a compound of the formula I according to claim 1, **characterised in that**
(a) a benzophenone compound of the formula II: 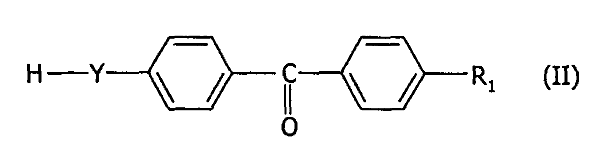 wherein Y is the oxygen atom or the sulphur atom and R₁ represents CN, CF₃ or SO₂CH₃.
is reacted with a D-xylose compound of the formula III: 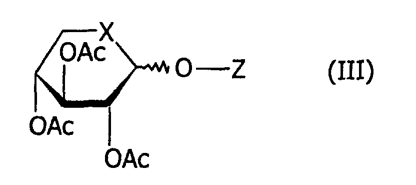 wherein
X is an oxygen atom or a sulphur atom,
Ac represents the acetyl group and Z represents an acetyl group or a trichloromethylimino group [-C(=NH)-CCl₃]
the reaction being conducted in a solvent and in the presence of a Lewis acid, in order to obtain after purification a compound of the formula I: 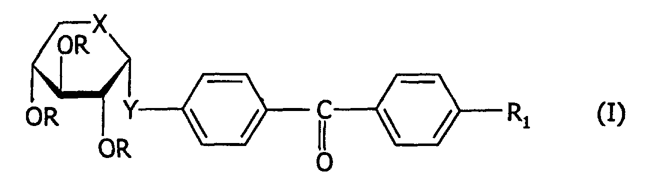 wherein
X, Y and R₁ retain the same significance as in the starting products and R represents an acetyl group.
(b) if necessary, the compound of the formula I, thus obtained and in which R is the acetyl group, is reacted with a base in order to replace the acetyl group with a hydrogen atom and to obtain the compound of the formula I in which R is a hydrogen atom, and
(c) if necessary, the compound of the formula I, thus obtained and in which R is a hydrogen atom, is reacted with a C₂-C₅ acid chloride or an anhydride of a C₂-C₅ aliphatic acid, in the presence of an aprotic base and in a solvent, in order to obtain the compound of the formula I in which R is an acyl group.

6. A pharmaceutical composition **characterised in that** it contains, in association with a physiologically acceptable excipient, a therapeutically effective quantity of at least one compound of the formula I according to claim 1.

7. A use of a product selected from the group consisting of the compounds of the formula I according to claim 1, for the preparation of an antiatheromatous drug intended for use in therapeutics vis-a-vis atherosclerosis.
